# EUROPEAN PATENT SPECIFICATION

(11) **EP 2 836 199 B1**
(45) Date of publication and mention of the grant of the patent: **19.06.2024**
(21) Application number: 13737670.3
(22) Date of filing: 09.04.2013
(51) Int. Cl.: A61K 9/00, A61K 9/06, A61K 47/10, A61K 47/14, A61K 47/20, A61K 31/565, A61P 35/00

(54) **FULVESTRANT FORMULATIONS**
FULVESTRANTFORMULIERUNGEN
FORMULATIONS DE FULVESTRANT

(30) Priority: 09.04.2012 IN 1418CH2012
(43) Date of publication of application: 18.02.2015
(73) Proprietor: Eagle Pharmaceuticals, Inc., Woodcliff Lake, NJ 07677 (US)
(72) Inventor: TEJA, Bulusu Bhanu, Hyderabad 500072 (IN); PALEPU, Nagesh R., Southampton, PA 18966-1166 (US)
(74) Representative: Carpmaels & Ransford LLP
(86) International application number: PCT/IN2013/000235
(87) International publication number: WO 2013/153559

(56) References cited:
- WO-A1-01/51056
- WO-A2-2009/111057

## Description

### CROSS-REFERENCE TO RELATED APPLICATION

This patent application claims priority to IN Provisional Patent Application No. 1418/CHE/2012, filed April 9, 2012, entitled "FULVESTRANT FORMULATIONS".

### BACKGROUND OF THE INVENTION

Fulvestrant injection for intramuscular administration is an estrogen receptor antagonist is marketed as FASLODEX. The chemical name is 7-alpha-[9-(4,4,5,5,5-penta fluoropentylsulphinyl) nonyl]estra-1,3,5-(10)- triene-3,17-beta-diol. The molecular formula is C₃₂H₄₇F₅O₃S and its structural formula is:

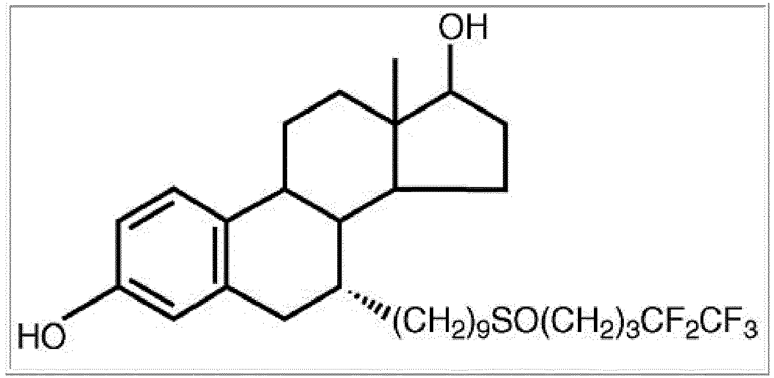

FASLODEX is supplied in sterile single patient pre-filled syringes containing 50-mg/ml fulvestrant either as 5 ml or 2.5 ml injections to deliver the required monthly dose. The current dosing regimen includes injecting 500mg (10ml injection). Most of the formulation contains castor oil, which results in an extremely painful injection. Typical side effects of castor oil and its derivatives are (a) skin and other irritation at the administration site; (b) allergic reaction; (c) gastrointestinal disturbances even though the product is not administered to the GI tract. Hence there is a need to find a more concentrated system which is free of castor oil, to allow low volume administration of medicament and reduction in pain.

WO 01/51056 discloses a novel sustained release pharmaceutical formulation adapted for administration by injection containing the compound 7 alpha -[9-(4,4,5,5,5-pentafluoropentylsulphinyl)nonyl]oestra-1,3,5(10)-triene-3,17 beta -diol, more particularly to a formulation adapted for administration by injection containing the compound 7 alpha -[9-(4,4,5,5,5-pentafluoropentylsulphinyl)nonyl]oestra-1,3,5(10)-triene-3,17 beta -diol in solution in a ricinoleate vehicle which additionally comprises at least one alcohol and a non-aqueous ester solvent which is miscible in the ricinoleate vehicle.

WO 2009/111057 discloses fulvestrant formulations suitable for intramuscular injection at concentration in excess of 40 mg/ml in the absence of castor oil and castor oil derivatives.

### SUMMARY OF THE INVENTION

According to the invention, the fulvestrant-containing compositions include a solvent selected from dimethyl sulfoxide (DMSO), glycofurol, N-methyl pyrrolidone, and mixtures thereof; an oil mixture selected from i) caprylic and capric triglycerides; and a sustained release member selected from benzyl benzoate, dihydrolipoic acid and benzyl alcohol; and the volume ratio of solvent: oil mixture : sustained release member is 1-2.5 : 0.9-2.5 : 0.9-2.5. The amount of fulvestrant included in the compositions is from 50 mg/ml to 150 mg/ml. Some aspects of the invention include fulvestrant-containing compositions for use in methods of treatment.

One of the advantages of the inventive compositions is that they have substantially improved long term stability when compared to currently available formulations. For example, the inventive fulvestrant compositions are substantially free of impurities after at least about 24 months at a temperature of from about 5 °C to about 25 °C. The inventive formulations are advantageously ready to use and preferably administered intramuscularly, but can be administered by suitable injection routes that are suitable for the fulvestrant active agent.

Another advantage of the inventive compositions is that they are substantially free or completely free of castor oil and castor oil derivatives.

### BRIEF DESCRIPTION OF THE DRAWINGS

Figure 1 is data corresponding to Example 2.
Figure 2 is data corresponding to Example 3.

### DETAILED DESCRIPTION OF THE INVENTION

Unless defined otherwise, all technical and scientific terms used herein have the same meaning as is commonly understood by one of ordinary skill in the art to which this invention belongs. In the event that there is a plurality of definitions for a term herein, those in this section prevail unless stated otherwise.

As used herein, RRT is calculated by dividing the retention time of the peak of interest by the retention time of the main peak. Any peak with an RRT <1 elutes before the main peak, and any peak with an RRT >1 elutes after the main peak.

For purposes of the present invention, "substantially free of impurities" shall be understood to include fulvestrant-containing compositions in which the amount of total impurities is less than about 5%, as calculated on a normalized peak area response ("PAR") basis as determined by high performance liquid chromatography ("HPLC") at a wavelength of 223nm, after a period of about 24 months at a temperature of from about 5°C to about 25°C. The amount of impurities is further calculated as being based upon the original amount fulvestrant (or salt thereof) being present in the composition or formulation.

As used herein, "substantially free", when referencing the amount of castor oil and castor oil derivatives in the fulvestrant-containing formulations described herein means not more than about 5%, preferably not more than about 4%, about 3%, about 2%, about 1%, about 0.5%, about 0.1%, and about 0.01% (100 ppm). More preferably, the fulvestrant-containing compositions include undetectable amounts of castor oil and castor oil derivatives with the use of standard analytic equipment, i.e. zero. Each of the % amounts being with reference to the complete formulation.

In accordance with one aspect of the invention, there are provided long term storage stable fulvestrant-containing compositions including:
a) fulvestrant at a concentration of from 50mg/ml to 150mg/ml;
b) a solvent selected from dimethyl sulfoxide (DMSO), glycofurol, N-methyl pyrrolidone, and mixtures thereof;
c) an oil mixture of caprylic and capric triglycerides,; and
d) a sustained release member selected from benzyl benzoate and benzyl alcohol; wherein said fulvestrant-containing composition having less than 5% total impurities, on a normalized peak area response ("PAR") basis as determined by high performance liquid chromatography ("HPLC") at a wavelength of 223nm, after at least 24 months of storage at temperatures of from 5°C to 25°C includes not more than 5% of castor oil and castor oil derivatives; wherein the volume ratio of solvent: oil mixture: sustained release member is 1-2.5: 0.9-2.5: 0.9-2.5.

The total impurities in the inventive compositions resulting from the degradation of the fulvestrant in the compositions is less than about 5% PAR as determined by HPLC at a wavelength of 223nm, after at least about 24 months of storage at a temperature of from about 5 °C to about 25 °C, and thus have long term stability for at least the same period of time or longer. Preferably, the fulvestrant-containing compositions demonstrate long term storage stability for at least about 36 months when stored under the conditions described herein. In one embodiment, the amount of total impurities in the inventive compositions resulting from the degradation of the fulvestrant is less than about 3% PAR as determined by HPLC at a wavelength of 223nm after at least about 24 months at a temperature of from about 5 °C to about 25 °C. Preferably, the amount of any individual degradant in the inventive compositions does not exceed 2% PAR as determined by HPLC at a wavelength of 223nm after storage periods of at least about 24 months at a temperature of from about 5°C to about 25°C.

In some aspects of the invention, the solvent is present in an amount of from about 20% (v/v) to about 40% (v/v). Preferably, the solvent is present in an amount of about 33% (v/v). In some aspects of the invention, the oil mixture is present in an amount of from about 20% (v/v) to about 50% (v/v). Preferably, the oil mixture is present in an amount of about 25% (v/v). In other embodiments, the sustained release member is present in an amount of from about 20% (v/v) to about 50% (v/v). Preferably, the sustained release member is present in an amount of about 42% (v/v). According to the invention the volume ratio of solvent: oil mixture: sustained release member is 1 to 2.5: 0.9 to 2.5: 0.9 to 2.5. Preferably, the volume ratio of solvent: oil mixture: sustained release member is about 1.3: about 1: about 1.7.

In some embodiments, the solvent is DMSO, glycofurol or N-methyl pyrrolidone. In other embodiments of the invention, however, the solvent is a mixture of DMSO and glycofurol. Within this aspect, the volume ratio of DMSO: glycofurol: oil mixture: sustained release member is about 0.9 to about 1.1: about 0.9 to about 1.1: about 0.9 to about 1.1: about 0.9 to about 1.1. Preferably, the volume ratio of DMSO: glycofurol: oil mixture: sustained release member is about 1: about 1: about 1: about 1.

In the invention, the oil mixture is a mixture of caprylic and capric triglycerides. The oil mixture in the fulvestrant-containing compositions according to several aspects of the invention can be mixtures of the fatty acid triglycerides such as the products marketed under the name MIGLYOL, i.e. MIGLYOL 810, 812, 818, 829 and 840. See the product information sheet for MIGLYOL. The oil mixture is caprylic and capric triglycerides, such as MIGLYOL 812, preferably including 50-64% C₈ and 30-45% C₁₀ fatty acids.

Without meaning to be bound by any theory or hypothesis, the member can have the effect of allowing for steady state concentrations to be reached, i.e. sustaining the release of fulvestrant and maintaining the duration of circulation in the blood stream for extended periods, such as at least about 15 days, and preferably at least about 28 days. In some aspects of the invention, the member is selected from pharmaceutically acceptable compounds known to increase the duration of circulation of a drug in the blood stream, such as benzyl benzoate, dihydrolipoic acid, benzyl alcohol and lipoic acid. The member in the fulvestrant-containing compositions according to several aspects of the invention is benzyl benzoate. In some aspects of the invention, the inventive compositions achieve steady-state concentrations of fulvestrant in vivo after the first to sixth dose, in other aspects steady state concentrations of fulvestrant are achieved after the fourth to sixth dose. Without meaning to be bound by any theory or hypothesis, attainment of steady state concentration in vivo for fulvestrant shall be understood to be a level sufficient to therapeutically control estrogen levels in the patient.

In some aspects of the disclosure the fulvestrant concentration in the compositions is from about 45 mg/ml to about 250 mg/ml. In the inventive compositions, the fulvestrant concentration is from 50 mg/ml to 150 mg/ml. Preferably, the fulvestrant concentration is from about 100 mg/ml to about 125 mg/ml. It will be understood that compositions containing any useful concentration within the ranges, i.e. 45, 50, 55, 60, 70, 75, 80, 90, 100, 125, 150 . . . 250 are contemplated. In other embodiments, the fulvestrant concentration in the composition is about 50 mg/ml. In other embodiments, the fulvestrant concentration is about 100 mg/ml. In alternative aspects of the disclosure, the fulvestrant is outside these ranges, but the amounts will be sufficient for single or multiple administrations of dosages generally regarded as effective amounts. In some aspects of the invention, pharmaceutically acceptable salts of fulvestrant are also contemplated.

The fulvestrant-containing compositions according to the invention may include at least one antioxidant that is pharmaceutically acceptable for use in human and veterinary formulations. The antioxidant is not limited to those currently regarded as safe by any regulatory authority. For example, the antioxidant can be selected from among lipoic acid, dihydrolipoic acid, methionine, sulpha-containing amino acids, acetone sodium bisulfate, propyl gallate, butylated hydroxytoluene ("BHT"), butylated hydroxyanisole ("BHA") and sodium formaldehyde sulfoxylate. Suitable antioxidant concentrations in the compositions can range from about 2.5 mg/mL to about 35 mg/mL, and preferably from about 5 mg/mL to about 20 mg/mL or from about 10 mg/mL to about 15 mg/mL. In some other embodiments, the concentration of the antioxidant in the fulvestrant-containing compositions is about 5 mg/mL.

Other additives which may be included in the compositions include polyethylene glycol (PEG), propylene glycol (PG), phospholipids, and poloxamers, including those products marketed under the name LUTROL. The molecular weight of the PEG will be within the range of pharmaceutically acceptable weights, although PEG 400 is preferred in many aspects of the invention.

In view of the foregoing, some preferred long term storage stable fulvestrant-containing compositions in accordance with the invention include:
I.
   a) fulvestrant at a concentration of from 50mg/ml to 150mg/ml;
   b) DMSO;
   c) a mixture of caprylic and capric triglycerides, i.e. MIGLYOL 812; and
   d) benzyl benzoate;
   wherein the DMSO is present in an amount of about 33% (v/v), the mixture of caprylic and capric triglycerides is present in an amount of about 25% (v/v), and the benzyl benzoate is present in an amount of about 42% (v/v); or
II.
   a) fulvestrant at a concentration of from 50mg/ml to 150mg/ml;
   b) DMSO;
   c) a mixture of caprylic and capric triglycerides;
   d) benzyl benzoate; and
   e) glycofurol;
wherein the volume ratio of DMSO: mixture of caprylic and capric triglycerides: benzyl benzoate: glycofurol is about 1: about 1: about 1: about 1.

Each of these compositions have the same stability described above, i.e. less than about 5% total impurities PAR as determined by HPLC at a wavelength of 223nm, after at least about 24 months of storage at a temperature of from about 5 °C to about 25 °C. Preferably, the compositions are stable for at least about 36 months of storage at a temperature of from about 5 °C to about 25 °C.

Another embodiment of the invention provides compositions for use in methods of treating a fulvestrant treatable condition in mammals. The methods include administering to a patient in need thereof an effective amount of one of the fulvestrant-containing compositions described herein. Since the active ingredient portion of the inventive composition is an FDA-approved drug, those of ordinary skill will recognize that the doses of fulvestrant employed in this aspect of the invention will be similar to those employed in any treatment regimens designed for fulvestrant as marketed under the trade name FASLODEX. See the patient package insert containing dosing information. The methods of treatment also include administering the inventive formulations for any purpose or physical condition for which fulvestrant has been indicated as being useful. Examples of fulvestrant treatable conditions include benign or malignant disease of the breast or reproductive tract. In some aspects the conditions are hormonal dependent.

In some aspects of the invention, the fulvestrant-containing compositions will be formulated for intramuscular injection, although other injection routes that are compatible with fulvestrant and the excipients are also contemplated as within the invention.

### EXAMPLES

The following examples serve to provide further appreciation of the invention but are not meant in any way to restrict the effective scope of the invention.

### EXAMPLE 1

Fulvestrant-containing compositions were prepared as follows:
Formulation A (Inventive) was prepared by dissolving 50 mg fulvestrant in a 1 mL solution including DMSO: glycofurol: MIGLYOL 812, i.e. a mixture of caprylic and capric triglycerides: benzyl benzoate in a volume ratio of 1:1:1:1.
Formulation B (Inventive) was prepared in the same manner as Formulation A, except that the fulvestrant concentration was 100 mg/mL.
Formulation C (Inventive) was prepared by dissolving 125 mg fulvestrant in a 1 mL solution including 33% (v/v) DMSO, 25% (v/v) MIGLYOL 812, and 42% (v/v) benzyl benzoate.
Formulation D (Inventive) was prepared by dissolving 100 mg fulvestrant in a 1 mL solution including 33% (v/v) DMSO, 25% (v/v) MIGLYOL 812, and 42% (v/v) benzyl benzoate.

The samples were maintained at 40 °C and analyzed periodically for drug content and total impurities at intervals indicated in Table 1 below. The results obtained are presented in Table 1.

**TABLE 1 - Stability of Fulvestrant**

| **Formulation** | **Temp.** | **Time Period** | **Conc. (mg/mL)** | **% of Initial** | **%of Total** |
|---|---|---|---|---|---|
| Formulation A - 50mg/mL | Initial | | 49.2 | 100 | 0.06 |
| | 40°C | 30 days | 49.0 | 100.4 | 0.15 |
| DMSO:Glycofurol:Miglyol 812:Benzyl benzoate (1:1:1:1) qs to 1mL | | 70 days | 49.0 | 100 | 0.15 |
| | | 168 days | 48.9 | 100 | 0.18 |
| Formulation B - 100mg/mL | Initial | | 101.8 | 100 | 0.34 |
| | 40°C | 1 month | 101.4 | 99.6 | 0.42 |
| DMSO:Benzyl benzoate:Miglyol 812:Glycofurol (1:1:1:1) qs to 1mL | | 2 months | 100.9 | 99.1 | 0.41 |
| | | 3 months | 100.8 | 99.0 | 0.44 |
| | | 6 months | 100.6 | 98.8 | 0.45 |
| Formulation C - 125mg | Initial | | 123.6 | 100 | 0.29 |
| | 40°C | 1 month | 123.6 | 100.0 | 0.31 |
| DMSO:Benzyl benzoate:Miglyol 812 (0.33:0.42:0.25) qs to 1mL | | 2 months | 122.8 | 99.4 | 0.32 |
| | | 3 months | 121.4 | 98.2 | 0.28 |
| | | 6 months | 121.4 | 98.2 | 0.28 |
| Formulation D-100mg | Initial | | 101.7 | 100 | 0.28 |
| | 40°C | 1 month | 100.8 | 99.1 | 0.30 |
| DMSO:Benzyl benzoate:Miglyol 812 (0.33:0.42:0.25) qs to 1mL | | 2 months | 100.6 | 98.9 | 0.32 |
| | | 3 months | 100.2 | 98.5 | 0.33 |
| | | 6 months | 100.1 | 98.4 | 0.34 |

| | | | | | |
|---|---|---|---|---|---|
| Note: In Table 1 the total % impurities include total contributions from peaks at various RRTs. | | | | | |

As shown in Table 1, the inventive fulvestrant formulations are very stable in solutions containing at least one solvent, an oil mixture, and a sustained release member. Table 1 shows that fulvestrant, when dissolved at a concentration of from 50 mg/mL to 125 mg/mL, in DMSO, benzyl benzoate and Miglyol 812, with or without glycofurol, had less than about 0.5% total impurities after at least 1 month, and at least as long as 6 months, storage at 40 °C.

The data presented in Table 1 translates to fulvestrant-containing compositions including at least one solvent, an oil mixture and a sustained release member having a shelf life of at least about 24 months at 5 °C or 25 °C. In fact, the inventive compositions are expected to be stable for at least 36 months under ambient storage conditions. In contrast, a sample related to FASLODEX requires refrigerated storage, and therefore, is not as stable as the inventive compositions and would not be suitable for long-term storage as described herein.

### EXAMPLE 2

Fulvestrant-containing compositions (Formulation E, Formulation F and Formulation G) were prepared as follows:
Formulation E (Control) was prepared as a control with recinolic acid, which is a major component of castor oil. Formulation E was prepared by dissolving 50 mg fulvestrant in a 1 mL solution including recinolic acid and PG in a molar ratio of 1:1 recinolic acid: PG.
Formulation F (Inventive) was prepared by dissolving 50 mg fulvestrant in a 1 mL solution including N-methyl pyrrolidone: glycofurol: MIGLYOL 812: benzyl benzoate in a volume ratio of 1:1:1:1.
Formulation G (Control) FASLODEX was obtained commercially from AstraZeneca, and includes fulvestrant at a concentration of 50 mg/mL in a solution including 10% (w/v) ethanol, 10% (w/v) benzyl alcohol, 15% (w/v) benzyl benzoate and castor oil added to 1mL.

The PK profiles of Formulation E, Formulation F, and Formulation G were evaluated in rabbits.

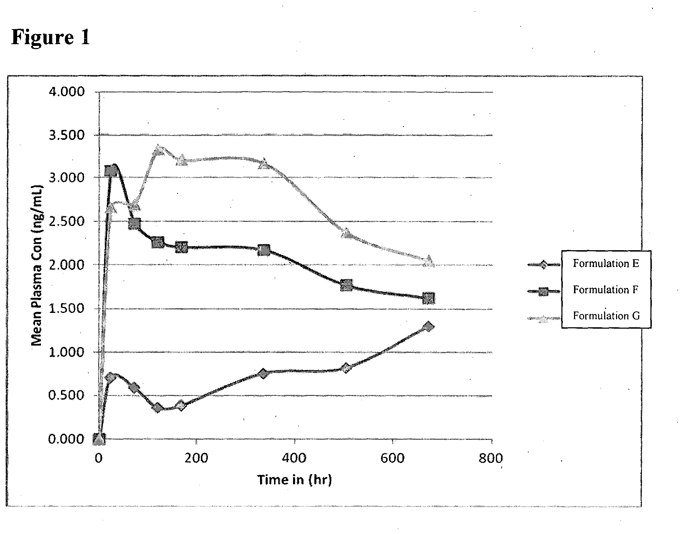

As shown in Figure 1, Formulation E showed lowest Cmax and the lowest mean plasma concentration. Inventive Formulation F exhibited a comparable PK profile to Formulation G. The PK profile of Formulation E, which shows the "low" levels, is inferior to that of inventive Formulation F and Formulation G. Accordingly, inventive Formulation F achieves a comparable PK profile to Formulation G, and is long term storage stable for at least 36 months under ambient conditions, without the inclusion of castor oil.

### EXAMPLE 3

Fulvestrant-containing compositions (Formulation A, Formulation H and Formulation G) were prepared as follows:
Formulation A (Inventive) was prepared as described above in Example 1.
Formulation H (Inventive) was prepared by dissolving 50 mg fulvestrant in a 1 mL solution including 33% (v/v) DMSO, 25% (v/v) MIGLYOL 812, and 42% (v/v) benzyl benzoate.
Formulation G (Control) was prepared as.described above in Example 2.

The PK profiles of Formulation A, Formulation H and Formulation G were evaluated in rabbits.

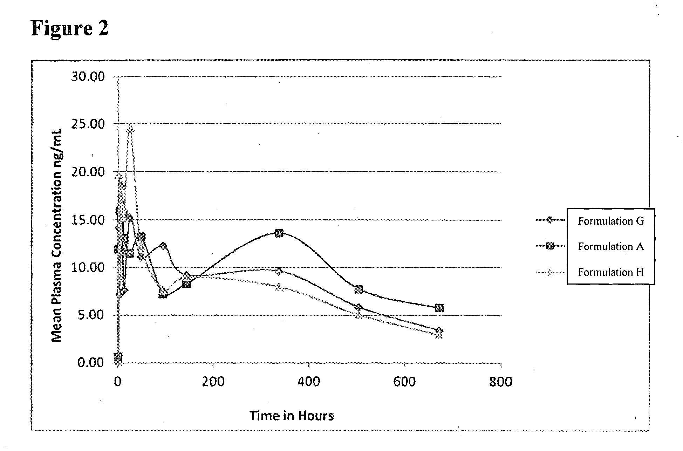

As shown in Figure 2, inventive Formulation A and inventive Formulation H exhibited PK profiles comparable to the PK profile of Formulation G. Accordingly, as shown in Table 1 and Figure 2, inventive Formulations A and H, are long term storage stable for at least 36 months under ambient conditions, and achieve a comparable PK profile to Formulation G, without the inclusion of castor oil.

### EXAMPLE 4

Fulvestrant-containing compositions (Formulation A, Formulation H and Formulation G) were prepared as follows:
Formulation A (Inventive) was prepared as described above in Example 1.
Formulation H (Inventive) was prepared as described above in Example 3.

Three separate Formulations G₁, G₂, and G₃ (Controls) were prepared in the same manner as Formulation G described above in Example 2.

The PK profiles of Formulation A, Formulation H, and Formulations G₁, G₂, and G₃ were evaluated in rabbits. The formulations were administered in an amount of 17 mg/kg rabbit body weight. Samples were drawn 28 days after administration. The PK data are presented in Table 2 below.

**TABLE 2**

| **Formulation** | **Cₘₐₓ (ng/mL)** | **AUC_{(0-dose)} (mg*h/L)** | **T1/2 (days)** |
|---|---|---|---|
| G₁ | 24.4 ± 7.2 | 6.8 ± 1.2 | 9 ± 3 |
| G₂ | 26.3 ± 10.8 | 5.3 ± 1.6 | 12 ± 8 |
| G3 | 28.7 ± 8.1 | 5.8 ± 2.9 | 20 ± 7 |
| A | 25.3 ± 11 | 6.3 ± 3.3 | 12 ± 7 |
| H | 27.6 ± 7.4 | 4.8 ± 1.2 | 38 ± 60 |

As shown in the table above, both inventive Formulations A and H exhibited similar PK profiles compared to Formulations G₁, G₂, and G₃.

### EXAMPLE 5

Additional PK studies were performed in rabbits to observe the formulations at steady state concentrations. Fulvestrant-containing compositions were prepared as follows:
Formulation G (Control) was prepared in the same manner as described above in Example 2.
Formulation A (Inventive) was prepared as described above in Example 1.
Formulation H (Inventive) was prepared as described above in Example 3.
Formulation B (Inventive) was prepared as described above in Example 1.
Formulation D (Inventive) was prepared as described above in Example 1.

The PK profiles of Formulation A, Formulation H, Formulation B, Formulation D and Formulation G were evaluated in rabbits. Doses were administered in an amount of 17 mg/kg rabbit body weight. The first dose was administered on day one, the second dose was administered on day 15, the third dose was administered on day 28, and subsequent doses (i.e. fourth, fifth, and sixth) were administered every month thereafter. Samples were drawn before each dose was administered. The PK data for the first dose, and doses four and five are reported in Table 3 below.

**TABLE 3**

| **Dose** | | **Formulation G** | **Formulation H** | **Formulation D** | **Formulation A** | **Formulation B** |
|---|---|---|---|---|---|---|
| **Dose 1** | **Cmax (ng/mL)** | 13.5 ±3.1 | 7.65 ± 4.61 | 5.37 ± 3.09 | 6.66 ± 2.28 | 3.37 ± 1.11 |
| | **AUC (h*ng/mL)** | 887 ± 227 | 582 ± 292 | 372 ± 137 | 518 ± 188 | 254 ± 81 |
| **Dose 4** | **Cmax (ng/mL)** | 49.3 ± 15 | 33.2 ± 4.9 | 25.3 ± 4.8 | 30.6 ± 12.0 | 18.9 ± 4.2 |
| | **AUC (h* ng/mL)** | 6146 ± 812 | 4897 ± 1030 | 3719 ± 780 | 4638 ± 1520 | 3260 ± 710 |
| **Dose 5** | **Cmax (ng/mL)** | 42.4 ± 9.9 | 40.6 ± 16.4 | 48.1 ± 25.3 | 37.1 ± 14.5 | 30.3 ± 4.2 |
| | **AUC (h*ng/mL)** | 6243 ± 982 | 6080 ± 1290 | 4985 ± 1260 | 5668 ± 2250 | 4065 ± 775 |

As shown in Table 3 above, inventive Formulations H, D, A and B achieved steady state concentrations comparable to Formulation G after four to five doses. Table 3 shows that comparable steady state concentrations to Formulation G can be obtained without including castor oil in the formulation. Accordingly, the inventive long term storage stable compositions made in accordance with the claimed invention achieved steady state concentrations comparable to Formulation G.

### EXAMPLE 6

Fulvestrant-containing compositions are prepared as follows:
Formulation I (Inventive) is prepared in the same manner as Formulation A in Example 1 above, except that the MIGLYOL 812 is replaced with MIGLYOL 810, i.e. a mixture of caprylic and capric triglycerides. The volume ratio is maintained at 1:1:1:1.
Formulation J (Inventive) is prepared in the same manner as Formulation H in Example 3 above, except that MIGLYOL 812 is replaced with MIGLYOL 810, i.e. a mixture of caprylic and capric triglycerides. The volume ratio is maintained at 33:25:42.
Formulations I and J have a shelf life of at least about 24 months at 5 °C or 25 °C, and are expected to be stable for at least 36 months under ambient storage conditions.

### EXAMPLE 7

Fulvestrant-containing compositions are prepared as follows:
Formulation K (Inventive) is prepared in the same manner as Formulation A in Example 1 above, except that the MIGLYOL 812 is replaced with MIGLYOL 818, i.e. a mixture of caprylic, capric and linoleic triglycerides. The volume ratio is maintained at 1:1:1:1.
Formulation L (Inventive) is prepared in the same manner as Formulation H in Example 3 above, except that MIGLYOL 812 is replaced with MIGLYOL 818, i.e. a mixture of caprylic, capric and linoleic triglycerides. The volume ratio is maintained at 33:25:42.
Formulations K and L have a shelf life of at least about 24 months at 5 °C or 25 °C, and are expected to be stable for at least 36 months under ambient storage conditions.

### EXAMPLE 8

Fulvestrant-containing compositions are prepared as follows:
Formulation M (Inventive) is prepared in the same manner as Formulation A in Example 1 above, except that the MIGLYOL 812 is replaced with MIGLYOL 829, i.e. a mixture of caprylic, capric and succinic triglycerides. The volume ratio is maintained at 1:1:1:1.
Formulation N (Inventive) is prepared in the same manner as Formulation H in Example 3 above, except that MIGLYOL 812 is replaced with MIGLYOL 829, i.e. a mixture of caprylic, capric and succinic triglycerides. The volume ratio is maintained at 33:25:42.
Formulations M and N have a shelf life of at least about 24 months at 5 °C or 25 °C.

### EXAMPLE 9

Fulvestrant-containing compositions are prepared as follows:
Formulation O (Inventive) is prepared in the same manner as Formulation A in Example 1 above, except that the MIGLYOL 812 is replaced with MIGLYOL 840, i.e. a mixture of propylene glycol dicaprylate and propylene glycol dicaprate. The volume ratio is maintained at 1:1:1:1.
Formulation P (Inventive) is prepared in the same manner as Formulation H in Example 3 above, except that MIGLYOL 812 is replaced with MIGLYOL 840, i.e. a mixture of propylene glycol dicaprylate and propylene glycol dicaprate. The volume ratio is maintained at 33:25:42.
Formulations O and P have a shelf life of at least about 24 months at 5 °C or 25 °C.

## Claims

1. A fulvestrant-containing composition comprising:
a) fulvestrant at a concentration of from 50 mg/ml to 150 mg/ml;
b) a solvent selected from the group consisting of dimethyl sulfoxide (DMSO), glycofurol, N-methyl pyrrolidone, and mixtures thereof;
c) an oil mixture of caprylic and capric triglycerides; and
d) a sustained release member selected from the group consisting of benzyl benzoate and benzyl alcohol;
wherein said fulvestrant-containing composition having less than 5% total impurities, on a normalized peak area response ("PAR") basis as determined by high performance liquid chromatography ("HPLC") at a wavelength of 223nm, after at least 24 months of storage at temperatures of from 5°C to 25°C includes not more than 5% of castor oil and castor oil derivatives;
wherein the volume ratio of solvent: oil mixture: sustained release member is 1-2.5 : 0.9-2.5 : 0.9-2.5.

2. The composition of claim 1, wherein the volume ratio of solvent: oil mixture: sustained release member is about 1.3: about 1: about 1.7.

3. The composition of any one of previous claim, wherein the solvent is present in an amount of about 33% (v/v), the oil mixture is present in an amount of about 25% (v/v) and the sustained release member is present in an amount of about 42% (v/v).

4. The composition of any one of previous claim, wherein the solvent is DMSO or the solvent is a mixture of DMSO and glycofurol.

5. The composition of any one of previous claim, wherein the sustained release member is benzyl benzoate.

6. The composition of claim 5, wherein the volume ratio of DMSO: glycofurol: oil mixture: sustained release member is about 0.9 to about 1.1: about 0.9 to about 1.1: about 0.9 to about 1.1: about 0.9 to about 1.1, preferably the volume ratio of DMSO: glycofurol: oil mixture: sustained release member is about 1: about 1: about 1: about 1.

7. The composition of any one of previous claim, wherein the fulvestrant concentration is from 100 mg/ml to 125 mg/ml, more preferably about 50 mg/ml, more preferably about 100 mg/ml.

8. The composition of any one of previous claim, further comprising at least one antioxidant selected from the group consisting of lipoic acid, dihydrolipoic acid, methionine, sulpha-containing amino acids, acetone sodium bisulfate, propyl gallate, butylated hydroxytoluene ("BHT"), butylated hydroxyanisole ("BHA") and sodium formaldehyde sulfoxylate.

9. The fulvestrant-containing composition according to claim 1, wherein said composition comprises:
a) fulvestrant at a concentration of from 50 mg/ml to 150 mg/ml;
b) DMSO;
c) a mixture of caprylic and capric triglycerides; and
d) benzyl benzoate;
wherein the DMSO is present in an amount of about 33% (v/v), the mixture of caprylic and capric triglycerides is present in an amount of about 25% (v/v), and the benzyl benzoate is present in an amount of about 42% (v/v);
said fulvestrant-containing composition having less than about 5% total impurities, on a normalized peak area response ("PAR") basis as determined by high performance liquid chromatography ("HPLC") at a wavelength of 223nm, after at least about 24 months of storage at temperatures of from about 5°C to about 25°C.

10. The fulvestrant-containing composition according to claim 1, wherein said composition comprises:
a) fulvestrant at a concentration of from 50 mg/ml to 150 mg/ml;
b) DMSO;
c) a mixture of caprylic and capric triglycerides;
d) benzyl benzoate; and
e) glycofurol;
wherein the volume ratio of DMSO: mixture of caprylic and capric triglycerides: benzyl benzoate: glycofurol is about 1: about 1: about 1: about 1;
said fulvestrant-containing composition having less than about 5% total impurities, on a normalized peak area response ("PAR") basis as determined by high performance liquid chromatography ("HPLC") at a wavelength of 223nm, after at least about 24 months of storage at temperatures of from about 5°C to about 25°C.

11. The fulvestrant-containing composition of any one of previous claim for use in a method of treating a fulvestrant treatable condition selected from benign or malignant disease of the breast or reproductive tract, comprising administering to a patient having said fulvestrant treatable condition an effective amount of said composition.

12. The fulvestrant-containing composition for use according to claim 11, wherein the method comprises administering said composition in an amount sufficient to maintain the duration of circulation in the blood stream for at least about 28 days.

## Patentansprüche

1. Fulvestranthaltige Zusammensetzung, umfassend:
a) Fulvestrant in einer Konzentration von 50 mg/ml bis 150 mg/ml,
b) ein aus der aus Dimethylsulfoxid (DMSO), Glycofurol, N-Methylpyrrolidon und Mischungen davon bestehenden Gruppe ausgewähltes Lösungsmittel,
c) eine Ölmischung aus Caprylsäure- und Caprinsäuretriglyceriden und
d) ein aus der aus Benzoesäurebenzylester und Benzylalkohol bestehenden Gruppe ausgewählter Bestandteil für eine verzögerte Freisetzung,
wobei die fulvestranthaltige Zusammensetzung nach mindestens 24 Monaten Lagerung bei Temperaturen von 5 °C bis 25 °C mit weniger als 5 % Gesamtverunreinigungen auf Basis einer normalisierten Peakflächenempfindlichkeit (Peak Area Response, "PAR"), bestimmt durch Hochleistungsflüssigchromatographie ("HPLC") bei einer Wellenlänge von 223 nm, nicht mehr als 5 % Rizinusöl und Rizinusölderivate enthält,
wobei das Volumenverhältnis von Lösungsmittel: Ölmischung: Bestandteil für eine verzögerte Freisetzung 1-2,5 : 0,9-2,5 : 0,9-2,5 beträgt.

2. Zusammensetzung nach Anspruch 1, wobei das Volumenverhältnis von Lösungsmittel: Ölmischung: Bestandteil für eine verzögerte Freisetzung etwa 1,3: etwa 1: etwa 1,7 beträgt.

3. Zusammensetzung nach einem der vorhergehenden Ansprüche, wobei das Lösungsmittel in einer Menge von etwa 33 Vol.-% vorhanden ist, die Ölmischung in einer Menge von etwa 25 Vol.-% vorhanden ist und der Bestandteil für eine verzögerte Freisetzung in einer Menge von etwa 42 Vol.-% vorhanden ist.

4. Zusammensetzung nach einem der vorhergehenden Ansprüche, wobei es sich bei dem Lösungsmittel um DMSO handelt oder es sich bei dem Lösungsmittel um eine Mischung von DMSO und Glycofurol handelt.

5. Zusammensetzung nach einem der vorhergehenden Ansprüche, wobei es sich bei dem Bestandteil für eine verzögerte Freisetzung um Benzoesäurebenzylester handelt.

6. Zusammensetzung nach Anspruch 5, wobei das Volumenverhältnis von DMSO: Glycofurol: Ölmischung: Bestandteil für eine verzögerte Freisetzung etwa 0,9 bis etwa 1,1: etwa 0,9 bis etwa 1,1: etwa 0,9 bis etwa 1,1: etwa 0,9 bis etwa 1,1 beträgt und das Volumenverhältnis von DMSO: Glycofurol: Ölmischung: Bestandteil für eine verzögerte Freisetzung vorzugsweise etwa 1: etwa 1: etwa 1: etwa 1 beträgt.

7. Zusammensetzung nach einem der vorhergehenden Ansprüche, wobei die Fulvestrantkonzentration 100 mg/ml bis 125 mg/ml, besonders bevorzugt etwa 50 mg/ml, besonders bevorzugt etwa 100 mg/ml beträgt.

8. Zusammensetzung nach einem der vorhergehenden Ansprüche, weiterhin umfassend mindestens ein aus der aus Liponsäure, Dihydroliponsäure, Methionin, sulphahaltigen Aminosäuren, Aceton-Natriumhydrogensulfat, Propylgallat, Butylhydroxytoluol ("BHT"), Butylhydroxyanisol ("BHA") und Natriumformaldehydsulfoxylat bestehenden Gruppe ausgewähltes Antioxidationsmittel.

9. Fulvestranthaltige Zusammensetzung nach Anspruch 1, wobei die Zusammensetzung Folgendes umfasst:
a) Fulvestrant in einer Konzentration von 50 mg/ml bis 150 mg/ml,
b) DMSO,
c) eine Mischung aus Caprylsäure- und Caprinsäuretriglyceriden und
d) Benzoesäurebenzylester,
wobei das DMSO in einer Menge von etwa 33 Vol.-% vorhanden ist, die Mischung aus Caprylsäure- und Caprinsäuretriglyceriden in einer Menge von etwa 25 Vol.- % vorhanden ist und das Benzoesäurebenzoat in einer Menge von etwa 42 Vol.-% vorhanden ist,
wobei die fulvestranthaltige Zusammensetzung nach mindestens etwa 24 Monaten Lagerung bei Temperaturen von etwa 5 °C bis etwa 25 °C weniger als etwa 5 % Gesamtverunreinigungen auf Basis einer normalisierten Peakflächenempfindlichkeit (Peak Area Response, "PAR"), bestimmt durch Hochleistungsflüssigchromatographie ("HPLC") bei einer Wellenlänge von 223 nm, enthält.

10. Fulvestranthaltige Zusammensetzung nach Anspruch 1, wobei die Zusammensetzung Folgendes umfasst:
a) Fulvestrant in einer Konzentration von 50 mg/ml bis 150 mg/ml,
b) DMSO,
c) eine Mischung aus Caprylsäure- und Caprinsäuretriglyceriden,
d) Benzoesäurebenzylester und
e) Glycofurol,
wobei das Volumenverhältnis von DMSO: Mischung aus Caprylsäure- und Caprinsäuretriglyceriden: Benzoesäurebenzylester: Glycofurol etwa 1: etwa 1: etwa 1: etwa 1 beträgt,
wobei die fulvestranthaltige Zusammensetzung nach mindestens etwa 24 Monaten Lagerung bei Temperaturen von etwa 5 °C bis etwa 25 °C weniger als etwa 5 % Gesamtverunreinigungen auf Basis einer normalisierten Peakflächenempfindlichkeit (Peak Area Response, "PAR"), bestimmt durch Hochleistungsflüssigchromatographie ("HPLC") bei einer Wellenlänge von 223 nm, enthält.

11. Fulvestranthaltige Zusammensetzung nach einem der vorhergehenden Ansprüche zur Verwendung in einem Verfahren zur Behandlung eines mit Fulvestrant behandelbaren Leidens ausgewählt aus benignen oder malignen Krankheiten der Brust oder des Fortpflanzungstraktes, umfassend die Verabreichung einer wirksamen Menge der Zusammensetzung an einen Patienten mit dem durch Fulvestrant behandelbaren Leiden.

12. Fulvestranthaltige Zusammensetzung zur Verwendung nach Anspruch 11, wobei das Verfahren die Verabreichung der Zusammensetzung in einer zur Aufrechterhaltung der Zirkulation im Blutstrom über mindestens etwa 28 Tage ausreichenden Menge umfasst.

## Revendications

1. Composition contenant du fulvestrant comprenant :
a) du fulvestrant à une concentration allant de 50 mg/ml à 150 mg/ml ;
b) un solvant sélectionné dans le groupe constitué par le diméthylsulfoxyde (DMSO), le glycofurol, la N-méthylpyrrolidone et des mélanges correspondants ;
c) un mélange d'huile de triglycérides capryliques et capriques ; et
d) un élément de libération prolongée sélectionné dans le groupe constitué par le benzoate de benzyle et l'alcool de benzyle ;
dans laquelle ladite composition contenant du fulvestrant possédant moins de 5 % d'impuretés totales, sur une base de réponse de surface de pic normalisée (« PAR ») telle que déterminée par chromatographie liquide haute performance (« HPLC ») à une longueur d'onde de 223 nm, après au moins 24 mois de stockage à des températures allant de 5 °C à 25 °C, ne comprend pas plus de 5 % d'huile de ricin et de dérivés d'huile de ricin ;
dans laquelle le rapport en volume de solvant : mélange d'huile : élément de libération prolongée est de 1-2,5 : 0,9-2,5 : 0,9-2,5.

2. Composition selon la revendication 1, dans laquelle le rapport en volume de solvant : mélange d'huile : élément de libération prolongée est d'environ 1,3 : environ 1 : environ 1,7.

3. Composition selon l'une quelconque des revendications précédentes, dans laquelle le solvant est présent en une quantité d'environ 33 % (v/v), le mélange d'huile est présent en une quantité d'environ 25 % (v/v) et l'élément de libération prolongée est présent en une quantité d'environ 42 % (v/v).

4. Composition selon l'une quelconque des revendications précédentes, dans laquelle le solvant est le DMSO ou le solvant est un mélange de DMSO et de glycofurol.

5. Composition selon l'une quelconque des revendications précédentes, dans laquelle l'élément de libération prolongée est le benzoate de benzyle.

6. Composition selon la revendication 5, dans laquelle le rapport en volume de DMSO : glycofurol : mélange d'huile : élément de libération prolongée est d'environ 0,9 à environ 1,1 : d'environ 0,9 à environ 1,1 : d'environ 0,9 à environ 1,1 : d'environ 0,9 à environ 1,1, de préférence le rapport en volume de DMSO : glycofurol : mélange d'huile : élément de libération prolongée est d'environ 1 : environ 1 : environ 1 : environ 1.

7. Composition selon l'une quelconque des revendications précédentes, dans laquelle la concentration de fulvestrant est de 100 mg/ml à 125 mg/ml, plus préférablement d'environ 50 mg/ml, plus préférablement d'environ 100 mg/ml.

8. Composition selon l'une quelconque des revendications précédentes, comprenant en outre au moins un antioxydant sélectionné dans le groupe constitué par l'acide lipoïque, l'acide dihydrolipoïque, la méthionine, des acides aminés contenant sulpha, l'acétone, le bisulfate de sodium, le gallate de propyle, l'hydroxytoluène butylé (« BHT »), l'hydroxyanisole butylé (« BHA ») et le sulfoxylate de formaldéhyde de sodium.

9. Composition contenant du fulvestrant selon la revendication 1, dans laquelle ladite composition comprend :
a) du fulvestrant à une concentration allant de 50 mg/ml à 150 mg/ml ;
b) du DMSO ;
c) un mélange de triglycérides capryliques et capriques ; et
d) du benzoate de benzyle ;
dans laquelle le DMSO est présent en une quantité d'environ 33 % (v/v), le mélange de triglycérides capryliques et capriques est présent en une quantité d'environ 25 % (v/v), et le benzoate de benzyle est présent en une quantité d'environ 42 % (v/v) ;
ladite composition contenant du fulvestrant possédant moins d'environ 5 % d'impuretés totales, sur une base de réponse de surface de pic normalisée (« PAR ») telle que déterminée par chromatographie liquide haute performance (« HPLC ») à une longueur d'onde de 223 nm, après au moins environ 24 mois de stockage à des températures allant d'environ 5 °C à environ 25 °C.

10. Composition contenant du fulvestrant selon la revendication 1, dans laquelle ladite composition comprend :
a) du fulvestrant à une concentration allant de 50 mg/ml à 150 mg/ml ;
b) du DMSO ;
c) un mélange de triglycérides capryliques et capriques ;
d) du benzoate de benzyle ; et
e) du glycofurol ;
dans laquelle le rapport en volume de DMSO : mélange de triglycérides capryliques et capriques : benzoate de benzyle : glycofurol est d'environ 1 : environ 1 : environ 1 : environ 1 ;
ladite composition contenant du fulvestrant possédant moins d'environ 5 % d'impuretés totales, sur une base de réponse de surface de pic normalisée (« PAR ») telle que déterminée par chromatographie liquide haute performance (« HPLC ») à une longueur d'onde de 223 nm, après au moins environ 24 mois de stockage à des températures allant d'environ 5 °C à environ 25 °C.

11. Composition contenant du fulvestrant selon l'une quelconque des revendications précédentes pour une utilisation dans un procédé de traitement d'une affection pouvant être traitée par du fulvestrant sélectionnée parmi une maladie bénigne ou maligne du sein ou de l'appareil génital, comprenant l'administration à un patient atteint de ladite affection pouvant être traitée par du fulvestrant d'une quantité effective de ladite composition.

12. Composition contenant du fulvestrant pour une utilisation selon la revendication 11, dans laquelle le procédé comprend l'administration de ladite composition en une quantité suffisante pour maintenir la durée de circulation dans le flux sanguin pendant au moins environ 28 jours.
